# EUROPEAN PATENT APPLICATION

(11) **EP 2 070 881 A1**
(43) Date of publication of application: **17.06.2009**
(21) Application number: 08020026.4
(22) Date of filing: 17.11.2008
(51) Int. Cl.: C02F 11/04

(54) **Method and apparatus for preparing material for microbiological fermentations**

(30) Priority: 16.11.2007 EP 07022319
(71) Applicant: APV Systems Ltd., Beehive Ring Road Gatwick West Sussex RH6 0PA (GB)
(72) Inventor: Skudder, Paul, John, West Sussex RH13 6PE (GB)
(74) Representative: Friese, Martin

(57) **Abstract**

The present invention relates to a method for preparing organic material for microbiological fermentations of municipal waste water sludge for reducing the waste water sludge and/or of cellulosic material for the production of biofuels, with at least one of the following steps:
- disintegrating and/or liquefying the organic and/or cellulosic material by using a jet pump (1) and/or
- creating a supersonic shock zone in a jet pump (1) the organic and/or cellulosic material is supplied to in order to disintegrate and/or liquefy the organic and/or cellulosic material and/or
- applying high pressure steam to an annular ring chamber (6) enclosing a tube (2) of a jet pump (1) and directing the steam into the tube (2) of the jet pump (1) thereby sucking organic and/or cellulosic material into the tube (2) and creating a supersonic shock zone (9) in the jet pump (1) in order to disintegrate and/or liquefy the organic and/or cellulosic material. The present invention also relates to an apparatus for preparing organic material for microbiological fermentations of municipal waste water sludge for reducing the waste water sludge and/or of cellulosic material for production of biofuels comprising a jet pump (1).

## Description

The present invention relates to a method and an apparatus for preparing material for microbiological fermentations of organic and/or cellulosic material in accordance with the preamble of claim 1 and claim 11, respectively.

Microbiological fermentations are used in many processes for example, the aerobic and an-aerobic digestion of municipal waste water and in the production of biofuels from cellulosic material.

The efficiency of the fermentation process is affected by the ability of the micro-organisms to access the organic matter due to the presence of aggregates and or solid material.

In an-aerobic digestion of waste water, a sludge forms in the bottom of the digester that must be removed, dried and disposed of at substantial cost. WO 01/16037 A1 discloses a method for mechanical disruption of the sludge using a homogeniser. A schematic diagram of this process is shown in Fig. 1. The whole disclosure of WO 01/16037 A1 is hereby incorporated by reference. The homogeniser disrupts the sludge into smaller aggregates that may be returned to the an-aerobic digester whereupon the bacteria can continue digestion. As a result additional methane gas is produced and the final volume of sludge that has to be removed is reduced.

The process is energy intensive in the electrical power required to operate the mechanical homogeniser but a net energy balance can be obtained by pre-concentration of the sludge by a minimum factor of 1.5 to reduce the volume of material passing through the homogeniser. The energy obtained by the conversion of the additional methane to electricity offsets the electrical requirements of the homogeniser.

WO 00/07946 A1 (EP 1102726 A1; Applicant Paradigm) discloses the use of a mechanical homogeniser to homogenise sludge from the upstream aerobic digesters in waste water treatment prior to entry to the an-aerobic digesters. In this instance, very high homogenisation pressures are required and it is necessary to add sodium hydroxide to lyse the sludge to assist in the disruption process. A schematic diagram of this process is shown in Fig. 2. The whole disclosure of WO 00/07946 A1 is hereby incorporated by reference.

Apart from the high energy consumption, a problem in both of the above described processes is rapid damage to the homogenisation valves by sand and other abrasive material in the waste water. This necessitates frequent and costly maintenance and replacement of the homogeniser valves. The object of the invention is to provide an apparatus and a method for microbiological fermentation in accordance with the preamble of the independent claims with an improved efficiency.

The object of the invention is achieved with the features of the independent claims. Preferred embodiments of the invention are disclosed in the dependent claims.

In accordance with an embodiment of the invention there is provided a method for preparing organic material for microbiological fermentations of municipal waste water sludge for reducing the waste water sludge and/or of cellulosic material for the production of biofuels, with at least one of the following steps:
- disintegrating and/or liquefying the organic and/or cellulosic material by using a jet pump and/or
- creating a supersonic shock zone in a jet pump the organic and/or cellulosic material is supplied to in order to disintegrate and/or liquefy the organic and/or cellulosic material and/or
- applying high pressure steam to an annular ring chamber enclosing a tube of a jet pump and directing the steam into the tube of the jet pump thereby sucking organic and/or cellulosic material into the tube and creating a supersonic shock zone in the jet pump in order to disintegrate and/or liquefy the organic and/or cellulosic material.

In accordance with the invention the municipal waste water sludge can be recirculated through the jet pump and returned to the aerobic or an-aerobic fermenter.

In accordance with the invention the organic material can comprise cellulosic material and/or liquefying of the cellulosic material can take place before fermenting the cellulosic material.

In accordance with the invention a jet pump can be used for liquefication of a slurry in water of precut or pulverised cellulosic material.

In accordance with the invention chemicals such as sodium hydroxide can be added to aid the disruption or liquefication process preferably before or during the disruption or liquefication step.

In accordance with the invention acid or acids can be added to enable acid hydrolysis during disintegration or liquifaction.

In accordance with the invention acid or acids can be added post liquefication to enable hydrolysis.

In accordance with the invention enzymes can be added to hydrolyse the cellulose.

In accordance with the invention inexpensive and readily available sources of cellulosic materials can be utilized, such as cut grass, wheat stalks, sugar cane waste and/or sugar beet waste products.

In accordance with the invention biofuel can be produced, for instance to be used for heating devices and/or internal combustion engines of motor vehicles.

In accordance with an embodiment of the invention there is provided an apparatus for preparing organic material for microbiological fermentations of municipal waste water sludge for reducing the waste water sludge and/or of cellulosic material for production of biofuels having a jet pump, especially a steam jet pump, wherein the steam jet pump preferably comprising an annular ring chamber the steam is directed into.

In accordance with the invention jet pump can comprise a tube and an annular ring chamber provided in the wall of the tube and a nozzle connecting the annular ring chamber with the inner space of the tube, wherein a pipe is connected to the annular ring chamber for introducing high pressure steam into the annular ring chamber, such that a supersonic shock wave is created in the material contained in the tube behind the exit of the nozzle into the tube if liquid containing organic material is sucked into the tube, wherein the supersonic shock wave preferably extends across the full cross section of the tube.

In accordance with the invention the jet pump can be adapted and arranged for liquefying pulverised cellulosic material and/or reducing waste water sludge.

In accordance with the invention the apparatus can be arranged and adapted for performing a method in accordance with the invention, wherein the apparatus preferably comprises screen means, aerobic digester means, an-aerobic digester means and/or fermentation means.

In accordance with the invention more than one jet pump can be used or comprised, wherein the jet pumps are preferably used or connected in series.

In the production of biofuels from cellulosic material, liquefication will greatly improve the accessibility of the cellulose to subsequent acid or enzymatic hydrolysis and potentially release more fermentable sugars for conversion to alcohol.

The use of a jet pump as detailed in EP 1 549 856 B1 and/or WO2006/010949 A1 can be used to substitute for the mechanical homogenisation of both aerobic and an-aerobic municipal waste water sludge with the benefit of reduced capital and operating cost whilst minimising mechanical damage resulting in longer operating times between maintenance of the disruption device. The whole disclosure of EP1549856B1 and WO2006/010949 A1 is hereby incorporated by reference.

The production of biofuel from sugar and wheat is well documented. However, diversion of food grade crops to biofuel will cause food prices to increase and a shortage of food. Other sources of fermentable substances must be found that would otherwise be considered a waste product. Such products include cellulosic material that once liquified and hydrolysed provides a source of fermentable sugar. Mechanical means of liquification are costly and equipment can be expensive in energy usage and in maintenance.

In order to be commercially successful, the costs of producing ethanol from cellulosic material must be less than oil. It is therefore advantageous to be able to utilize inexpensive and readily available sources of cellulosic material such as cut grass, wheat stalks, sugar cane waste and/or sugar beet waste products.

A jet pump as detailed in EP 1 549 856 B1 and/or W02006/010949 A1 can similarly be applied to liquefy suspensions of cellulosic products in water such as cut grass and other products such as wheat stalks, sugar cane waste and sugar beet waste. Indeed, any cellulosic product. The resultant liquor may then be either enzymatically or chemically hydrolysed to fermentable sugars.

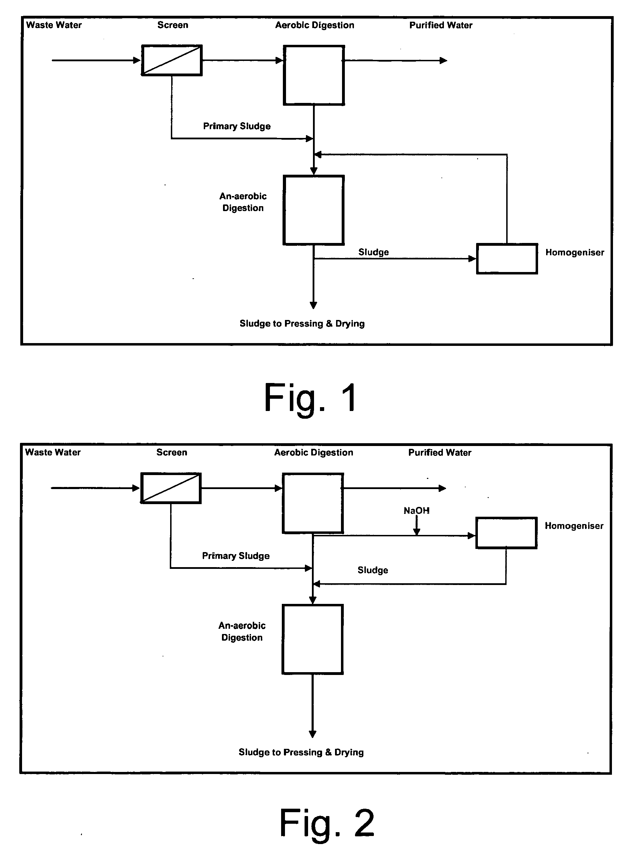

Fig. 1 is a schematic sketch of an apparatus and/or a method as disclosed in WO 01/16037 A1.
Fig. 2 is a schematic sketch of an apparatus and/or a method as disclosed in WO 00/07946 A1.
Fig. 3 is a schematic sketch of an apparatus and/or a method in accordance with a first embodiment of the invention.
Fig. 4 is a schematic sketch of an apparatus and/or a method in accordance with a second embodiment of the invention.
Fig. 5 is a schematic cross-section of a jet jump to be used in accordance with the invention and to be included into the apparatus in accordance with the invention.
Fig. 6 is a schematic cross-section of another jet jump to be used in accordance with the invention and to be included into the apparatus in accordance with the invention.
Fig. 7 is a schematic sketch of an apparatus and/or a method in accordance with a third and preferred embodiment of the invention.
Fig. 8 is a schematic sketch of an apparatus and/or a method in accordance with a fourth embodiment of the invention.
Fig. 9 is a schematic sketch of an apparatus and/or a method in accordance with a fifth embodiment of the invention.
Fig. 10 is a schematic sketch of an apparatus and/or a method in accordance with a sixth embodiment of the invention.

The following reference numerals are used in the specification of the present invention:
- 1: jet pump
- 2: tube
- 3: tube inlet
- 4: tube outlet
- 5: pipe
- 6: annular ring chamber
- 7: nozzle
- 8: mixing chamber
- 9: condensation shock wave
- 101: jet pump
- 102: tube
- 103: tube inlet
- 104: tube outlet
- 105: pipe
- 106: annular ring chamber
- 107: nozzle
- 108: mixing chamber
- 109: condensation shock wave
- 110: annular ring chamber
- 111: nozzle
- 112: annular ring chamber
- 113: pipe
- 201: screen means
- 202: aerobic digestion means
- 203: an-aerobic digestion means
- 204: filter means

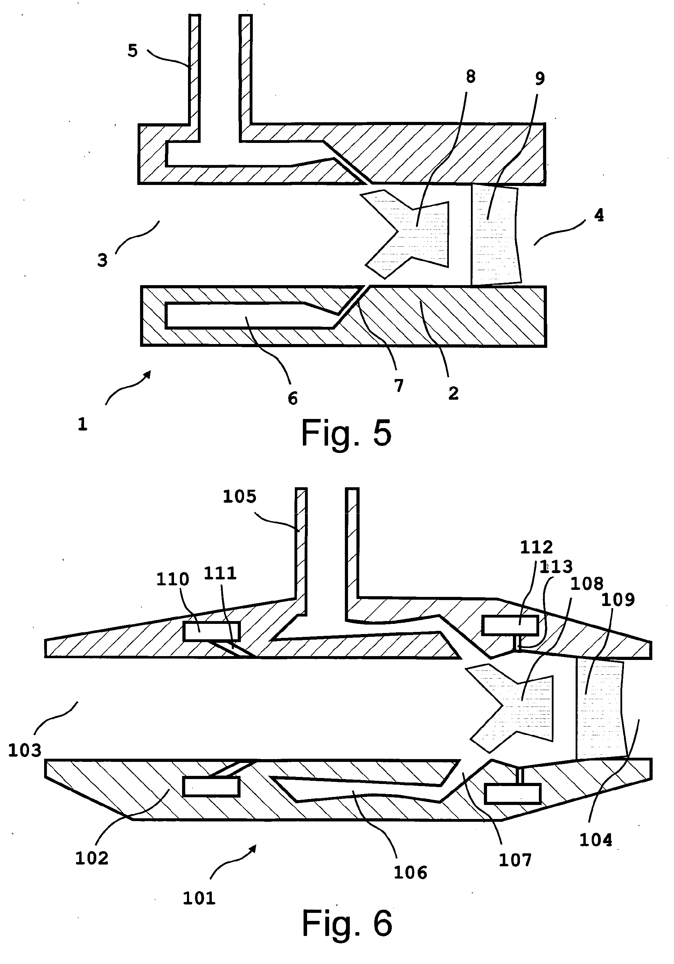

The jet pump to be used for the method of the invention and to be included into the apparatus of the invention is of a very specific type as for instance detailed in EP 1 549 856 B1 and/or WO2006/010949 A1. Schematic cross-sections of such jet pumps are shown in and described in connection with Figs. 5 and 6.

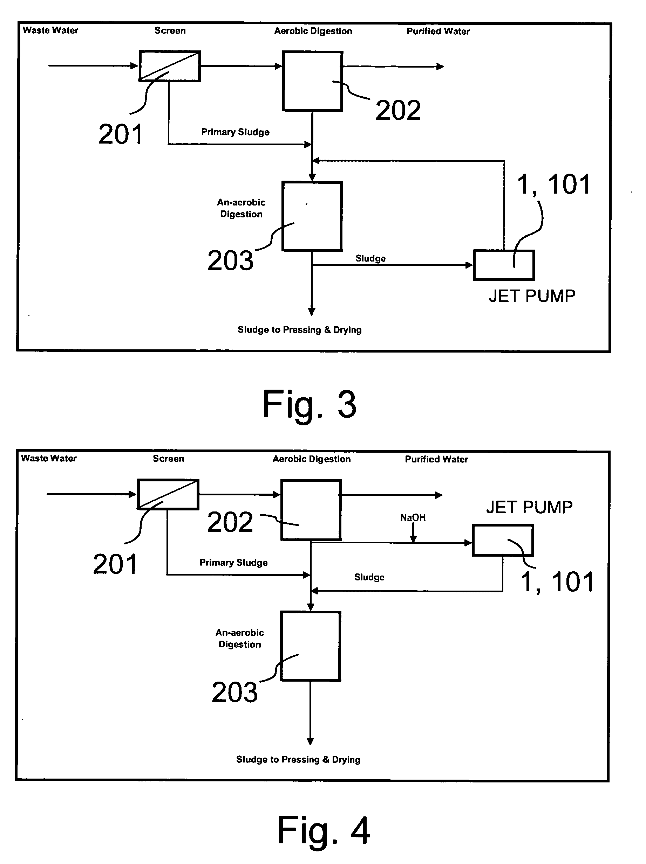

Fig. 3 is a schematic diagram of a method and apparatus for an-aerobic digestion of waste water. The mechanical disruption of a sludge forms in the bottom of the digester is made be means of a steam jet pump as shown in Fig. 5 and/or 6.

Waste water is provided to screen means 201. The liquid is fed to aerobic digestion means having as output purified water and sludge. The primary sludge of the screen means 201 and the sludge of the aerobic digester means 202 are fed to an an-aerobic digester means 203. Some sludge output of the an-aerobic digester means 203 is fed into the jet pump 1, 101. The disintegrated and/or liquefied sludge is recirculated to the inlet of the an-aerobic digesting means 203. The remainder output of the an-aerobic digester means will be pressed and dried in the usual manner. The provision of the steam jet pump 1, 101 instead of a homogenizer as known from the prior art mentioned above has the advantage that the wear and the costs are dramatically reduced.

In accordance with an alternative the jet pump 1, 101 could also be provided in addition or alternatively before the inlet of the an-aerobic digester means 203 such that all sludge to be fed into the an-aerobic digester means has to pass the steam jet pump 1, 101. Alternatively or in addition a steam jet pump 1, 101 as shown in and described in connection with Fig. 5 or Fig. 6 can be provided before the aerobic digester means 202 and/or the screen means 201 such that all material to be fed therein has to pass the steam jet pump 1, 101.

The jet pump 1, 101 can also potentially be used for treating or processing the aerobic sludge before it is entering the an-aerobic digester means.

In accordance with the invention the jet pump 1, 101 may be used for recirculation the sludge around the aerobic digester means and/or the an-aerobic digester means.

According to the invention the jet pump 1, 101 can also be used in combination with mechanical homogenisation, wherein the jet pump 1, 101 is to be placed before the homogeniser in order to protect the homogeniser getting damages by the sludge containing sand and grit. According to the invention the primary sludge can be treated only. It is clear that the sludge may be treated in addition thereafter by a jet pump 1, 101.

Fig. 4 is a schematic diagram of a method and apparatus for an-aerobic digestion of waste water in accordance with another embodiment of the invention. Same components as shown in Fig. 3 have same reference numerals. In the following only the differences to the embodiment of Fig. 3 will be described and reference is made to the description of the embodiment of Fig. 3. The output of the an-aerobic digester means 203 is not fed to the steam jet pump, but some of the sludge output of the aerobic digester 202 is fed into the steam jet pump 1, 101. The pH of the sludge may be increased before the treatment with the steam jet pump, for instance by adding NaOH. The disintegrated and/or liquefied sludge is then fed to the an-aerobic digester means 203.

The steam jet pump 1, 101, as shown in and described in connection with Fig. 5 and 6, is connected such that liquefying of the cellulosic material takes place before fermenting the cellulosic material.

Fig. 5 shows a schematic cross-section of such a jet pump. The jet pump 1 comprises a tube 2 with a tube inlet 3 and a tube outlet 4. In the wall of tube 2 there is provided an annular ring chamber 6 pipe 5 is connected to. The annular ring chamber 6 is connected to the inner space of tube 2 by nozzle 7.

In operation steam is pressed with a high pressure into pipe 5 and distributed in the annular ring chamber 6. From the annular ring chamber 6 the steam is guided through nozzle 7 into tube 2, thereby sucking liquid containing organic material into tube 2. The organic and/or cellulosic material will be sucked into the tube due to the condensation of the steam into the liquid. There is a reduction in volume as the steam condenses creating a pressure drop in the tube that sucks the material into the tube.

The area in the tube adjacent to the exit of nozzle 7 is functioning as a mixing chamber 8.

In operation high pressure steam is directed into pipe 5, whereby liquid is sucked into the hollow tube 2 via tube inlet 3, typically with a velocity of 8 to 10 m/s. In the mixing chamber 8 the liquid is mixed with the steam thereby creating a supersonic shockwave 9 because of the condensation of the steam and the high momentum of the steam exiting nozzle 7. At the exit of nozzle 7 there are supersonic velocities of a mix of steam and water droplets of about 800 m/s. At the tube outlet 8, the velocity will drop again to velocities of about 8 to 10 m/s.

It is clear that the mixing chamber 8 and the supersonic shockwave 9 as shown in Figs. 5 and 6 are only indications where the mixing takes place and shockwave occurs in the tube. It is not necessary that there are any means inserted into the tube in order to provide the mixing of the liquid with the steam and the generation of the supersonic shockwave. These effects are occurring because of the provision of the annular ring chamber 6 and the nozzle 7 directing steam into the liquid sucked into the tube.

The high velocities of the water droplets containing the organic material cause a physical disintegration and/or liquefication of the organic material. Any sludge agglomerates are physically mascerated to increase the efficiency of the an-aerobic digestion process of the invention by making the smaller agglomerates more accessible to the bacteria. As a result in the method and apparatus of the invention there is less digester sludge to be disposed of.

In accordance with the invention nozzle 7 can be an annular ring or a series of pipes distributed along the extension of the annular ring chamber and/or the circumference of the tube. According to an embodiment of the invention having a nozzle comprising pipes, the pipes may be oriented in angle in order to create a vortex in the tube.

Fig. 6 shows another embodiment of a jet pump 101 mainly corresponding to the jet pump 1 shown in Fig. 5. Similar parts have been designated with reference numerals increased by 100. In the following the additional components and/or features are described. For the corresponding features and components reference is made to the description of jet pump 1 as shown in Fig. 5.

The form of annular ring chamber 106 and nozzle 106 has been optimized in order to create a supersonic shock zone 109. There are annular ring chambers 110, 112 with pipes and/or nozzles 111, 113 through which additives or additions can be supplied into tube 102. The annular ring chambers 110, 112 have respective connections (not shown) for supplying the materials to be added, such as acids and/or enzymes to be used for the hydrolyse of complex polysaccharides.

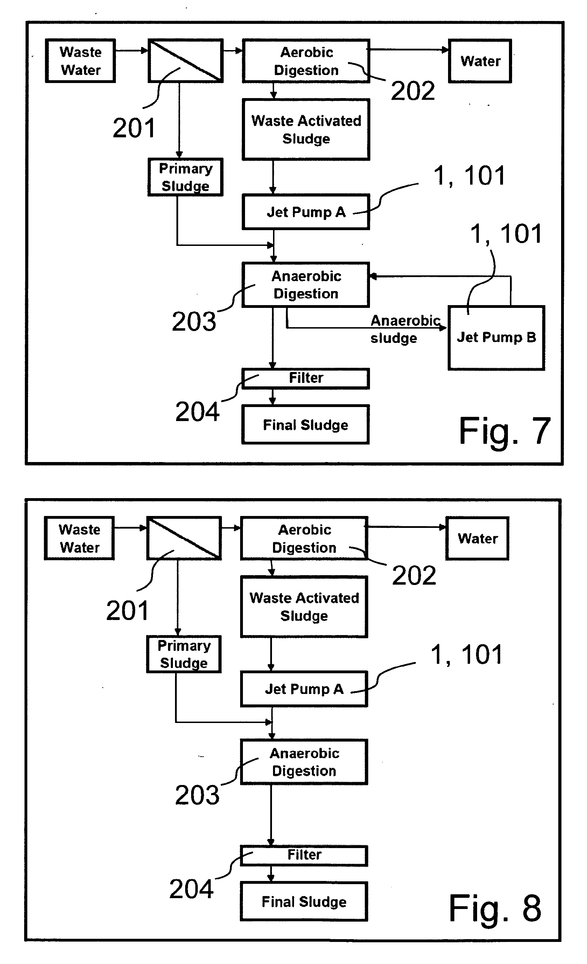

Figs. 7 to 10 are further embodiments of the invention mainly corresponding to the embodiments as shown in and described in connection with Figs. 3 and 4. The same reference numerals are used to designate similar or identical components. In the following only the differences are described. In the embodiments of Figs. 7 to 10 there are filter means 204 provided to filter the material exiting the an-aerobic digester means 203.

According to the embodiment of Fig. 7 there are two jet pumps 1, 101 provided as shown in and described in connection with Fig. 5 and 6.

Jet pump A is provided between the outlet of the aerobic digester means 202 where the sludge is exiting and the inlet an-aerobic digester means 203. The primary sludge from the screen filter means 201 is fed directly into the inlet of the an-aerobic digester means 203 bypassing jet pump A. Alternatively the primary sludge of the screen filter means may be directed through jet pump A or a further jet pump (not shown).

Jet pump B is arranged and connected in order to treat and recirculate the an-aerobic sludge exiting the an-aerobic digester means to the inlet of the an-aerobic digester means.

Fig. 8 is an embodiment of the invention where jet pump B of the embodiment of Fig. 7 is omitted.

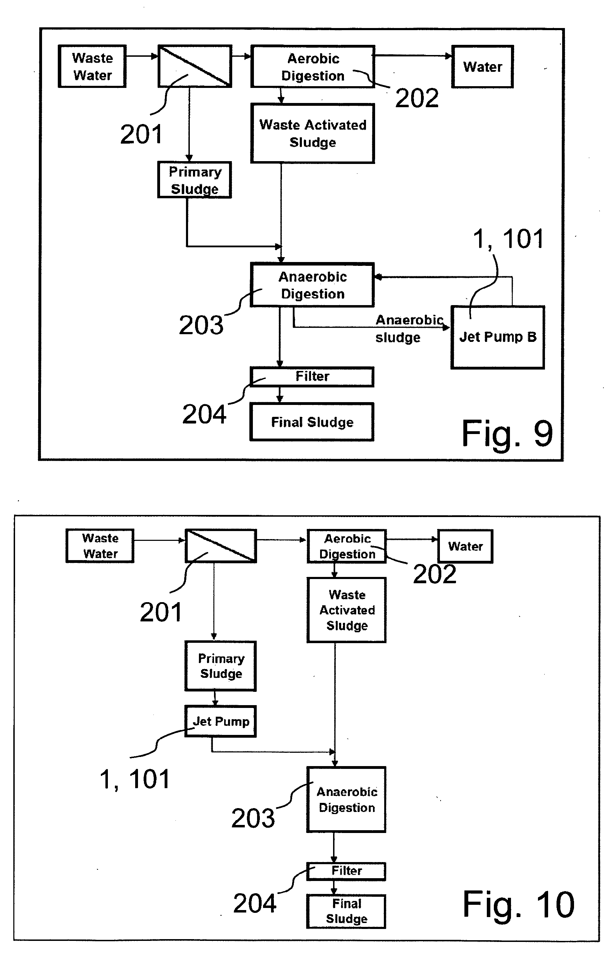

Fig. 9 is an embodiment of the invention where jet pump A of the embodiment of Fig. 7 is omitted.

Fig. 10 is an embodiment where a jet pump 1, 101 as shown in and described in connection with Fig. 5 or 6 is provided between the outlet of screen filter means 201 and the inlet of the an-aerobic digester means 203 in order to treat the primary sludge of the screen filter means 201 before entering the an-aerobic digester means.

In accordance with the invention embodiments comprising the use of a jet pump in any of the positions as shown in Fig. 7 to 10 are envisaged, either alone or in any possible combination. For instance the provision of three jet pumps at the position as shown in Fig. 8, as shown in Fig. 9 and as shown in Fig. 10 is possible.

In accordance with the invention, in addition and preferably before or better instead of the homogenizer as used in the prior art methods and apparatuses, a jet pump creating a supersonic shock wave to disintegrate and/or liquefy the organic material and/or sludge is used.

Accordingly a jet pump as described above and as known in the prior art for other purposes and being incorporated by reference is to be used instead of the homogenizer. In other applications the jet pump can be used in the process before the homogenizer.

A large proportion of the world biofuel is currently produced from primary materials such as sugar and corn. However, the use of primary food products is inflating the price of food. For this reason, attention is being placed on the economic production of biofuel from secondary plant materials such as grass, sugar beet waste and corn stalks.

The waste material must be reduced in size and preferably completely mascerated in order to solubilise the constituent cellulose. Once solubilised, the cellulose must be hydrolysed to convert it to carbohydrates that can then be fermented using yeast or bacteria to convert the carbohydrate to ethanol or butanol. The dilute alcohol stream that is produced is then evaporated and then distilled before final drying using a molecular sieve.

The use of the steam jet pump which is schematically shown for instance in Fig. 5 or 6 offers a low cost means of rapidly solubilising the cellulose following mechanical disintegration using for example a chopping device. The steam jet pump can also contribute to hydrolysis of the cellulose through the heating effect and by addition of acid prior to entering the pump.

An overview of known methods for producing biofuel is disclosed at the homepage of the U.S. department of energy, Energy Efficiency and renewable Energy, is section BIOMASS PROGRAM: INFROMATION RESOURCES:
http://www1.eere.energy.gov/biomass/abcs_biofuels.html

The whole disclosure thereof is hereby incorporated by reference.

Firstly complex polysaccharides are to be converted into simple sugars by hydrolysis. Acids and enzymes are used to catalyse the hydrolysis. Secondly sugars are converted to ethanol by fermentation caused by yeast or bacteria which fed on the sugars. Thereby ethanol and carbon oxide is produced out of the sugar.

According to the method and apparatus of the present invention the biomass containing polysaccharide is treated with a jet pump creating a supersonic shock wave. Thereby the biomass is liquefied and the polysaccharide may be at least partly hydrolysed.

Accordingly the invention also relates to a method and an apparatus for microbiologic fermentation of cellulosic material, wherein a jet pump for liquefying pre-cut or pulverised cellulosic material is used.

## Claims

1. Method for preparing organic material for microbiological fermentations of municipal waste water sludge for reducing the waste water sludge and/or of cellulosic material for the production of biofuels,
**characterized by**
- disintegrating and/or liquefying the organic and/or cellulosic material by using a jet pump and/or
- creating a supersonic shock zone in a jet pump the organic and/or cellulosic material is supplied to in order to disintegrate and/or liquefy the organic and/or cellulosic material and/or
- applying high pressure steam to an annular ring chamber enclosing a tube of a jet pump and directing the steam into the tube of the jet pump thereby sucking organic and/or cellulosic material into the tube and creating a supersonic shock zone in the jet pump in order to disintegrate and/or liquefy the organic and/or cellulosic material.

2. Method in accordance with claim 1, wherein the municipal waste water sludge is recirculated through the jet pump and returned to the aerobic or an-aerobic fermenter.

3. Method in accordance with claim 1, wherein the organic material comprises cellulosic material and wherein liquefying of the cellulosic material takes place before fermenting the cellulosic material.

4. Method in accordance with any of the preceding claims, wherein a jet pump is used for liquefication of a slurry in water of precut or pulverised cellulosic material.

5. Method in accordance with any of the preceding claims, wherein chemicals such as sodium hydroxide are added to aid the disruption or liquefication process preferably before or during the disruption or liquefication step.

6. Method in accordance with any of the preceding claims, wherein acid or acids are added to enable acid hydrolysis during disintegration or liquifaction.

7. Method in accordance with any of the preceding claims, wherein acid or acids are added post liquefication to enable hydrolysis.

8. Method in accordance with any of the preceding claims, wherein enzymes are added to hydrolyse the cellulose.

9. Method in accordance with any of the preceding claims, wherein inexpensive and readily available sources of cellulosic materials are utilized, such as cut grass, wheat stalks, sugar cane waste and/or sugar beet waste products.

10. Method in accordance with any of the preceding claims, wherein biofuel is produced, for instance to be used for heating devices and/or internal combustion engines of motor vehicles.

11. Apparatus for preparing organic material for microbiological fermentations of municipal waste water sludge for reducing the waste water sludge and/or of cellulosic material for production of biofuels **characterized by** a jet pump.

12. Apparatus in accordance with the preceding claim wherein the jet pump comprises a tube and an annular ring chamber provided in the wall of the tube and a nozzle connecting the annular ring chamber with the inner space of the tube, wherein a pipe is connected to the annular ring chamber for introducing high pressure steam into the annular ring chamber, such that a supersonic shock wave is created in the material contained in the tube behind the exit of the nozzle into the tube if liquid containing organic material is sucked into the tube, wherein the supersonic shock wave preferably extends across the full cross section of the tube.

13. Apparatus in accordance with any of the two preceding claims wherein the jet pump is adapted and arranged for liquefying pulverised cellulosic material and/or reducing waste water sludge.

14. Apparatus in accordance with any of the three preceding claims, being arranged and adapted for performing a method in accordance with any of the preceding method claims, wherein the apparatus preferably comprises screen means, aerobic digester means, an-aerobic digester means and/or fermentation means.

15. Method or apparatus in accordance with any of the preceding claims, wherein more than one jet pump is used or comprised, wherein the jet pumps are preferably used or connected in series.
